# EUROPEAN PATENT APPLICATION

(11) **EP 3 056 080 A1**
(43) Date of publication of application: **17.08.2016**
(21) Application number: 15154573.8
(22) Date of filing: 10.02.2015
(51) Int. Cl.: A01H 1/06, A01H 1/02

(54) **Method of producing hybrid F1 seed of angiosperm plants**

(71) Applicant: Vyzkumne centrum SELTON, s.r.o., 25084 Sibrina (CZ)
(72) Inventor: Sedlacek, Tibor, 25084 Sibrina (CZ)
(74) Representative: Hartvichova, Katerina

(57) **Abstract**

The present invention provides a method of producing hybrid F1 seed of an angiosperm plant, which contains the following steps:
- a maternal line is prepared by inactivation of a gene involved in the synthesis of sporopollenin and by mutation of acetolactate synthase gene resulting in resistance to herbicides of the acetolactate synthase inhibitor group;
- a paternal line is prepared, which is a fertile line without resistance to herbicides of the acetolactate synthase inhibitor group;
- subsequently, hybrid F1 seeds are produced by mixing the seed of the sterile herbicide-tolerant maternal line and the fertile herbicide-sensitive paternal line in a 95:5 ratio and by subsequent sowing this mixture of seeds of the maternal line and paternal line; and
- after the flowering period of the vegetation of mixture of the maternal line and paternal line, the plants are treated with a herbicide of the acetolactate synthase inhibitor group, thereby killing the herbicide-sensitive paternal line plants and leaving the herbicide-resistant maternal line plants producing pure fertile hybrid F1 seed;
- whereas the sterile maternal line is maintained by spraying with 4-oxo-6-octadecane-pyran-2-olate in the phase in which pollen tetrads are formed.

SEQUENCE LISTING

<110>  Vyzkumne centrum Selton, s.r.o.

<120>  Method of producing hybrid F1 seed of angiosperm plants

<130>  P

<160>  5

<170>  PatentIn version 3.5

<210>  1
<211>  1480
<212>  DNA
<213>  Triticum aestivum

<400>  1
catggcgagc atcccctatt gaagcgaagc acccatcacg cacacagtga aatcacagga      60

gcggagaact tgtaaacctg aagcggaagc aagcaaggag cgcctgtgag gcagcgatgt     120

ctggccttgg tggactcggg agcatcggcg ggggcggcaa cggcataagc cacggctcgg     180

ggggcaaggc caagctgctc gcactcggca agggtctccc tgaacaagtt ctaccccagg     240

agaagctcgt cgagacctac ctccaggaca ccagctgcga cgatcccgcc accagggcca     300

agctcgaacg cctctgcaag accacaacag tgaggacaag gtacactgtc atgtcgaagg     360

agctcctgga tgaataccca gaattgaaga cagaaggctc tccaacattg acgccacggc     420

ttgacatctg caatgccgcg gtgcttgaac ttggtgctgc tgcagctcgt gccgcccttg     480

gtgagtgggg ccgtccagca gctgatatta cccacctggt ctacatctcc tccagcgagc     540

ttcgcctccc agggggtgat cttttcctgg caacccgtct tggcctctct ccaaacacag     600

tgcgcacatc ccttctcttc cttggctgct caggtggtgc tgccgccctc cgcactgcca     660

aggacattgc agagaacaac ccagggagcc gtgtccttgt aatagcggcg gagacaactg     720

tgttgggctt ccggccgcca agccctgatc gtccttatga cctcgttggt gctgctctgt     780

ttggtgatgg tgcatcagct gcaattattg gagcaagccc tatcaaagca gaagaggatc     840

ctttcttgga gcttgagttc tcaacccagg agttcctacc agggacagac aaggtaattg     900

atggcaagat ctcagaggaa gggattaact tcaaactggg gcgtgatttg cctgaaaaga     960

ttgaaagccg catagaaggc ttctgcagga tactcatgga caaggttggg ataaaggagt    1020

tcaatgacgt attttgggct gtgcatcccg gcggaccagc aatattgaac aggctggagg    1080

tttgtcttga acttgagcca gataaactca agatcagtag aaaggccctg atgaactatg    1140

ggaatgtgag cagcaacaca gtcttctatg tgctggagta tttgagggat gaattgaaga    1200

aaggggcaat aagggaagag tggggattga tcttggcttt tggcccaggc atcacatttg    1260

aaggaatgct agttcggggc attaactgaa attgaaggag atccaagtag gttactacca    1320

gatacatgga aagatcaaaa acaagcttta tatggcacag tagcatttca caattataat    1380

tatacagtgt aaggatagca attcgcggct ataattatat actgtaagaa tatcataagc    1440

atagcgtctt gatgatttct gttcactaca tactcagcac                          1480

<210>  2
<211>  1496
<212>  DNA
<213>  Hordeum vulgare

<400>  2
gagcgaaatc acaggagcgg agaaattcta aacctgaggc agaaggaagc aacgagcgtc      60

tgtgagtcag cgatgtctgg ccttggaagc atcggcgggg gcggcaacgg cagaagccat     120

ggttcagggg gcaaggccaa gctgctcgcg ctcggcaagg gtctccctga gcaagttcta     180

ccccaggaga agctcgtcga gacctacctc caggacacca gctgcgacga tcccgccacc     240

agggccaagc tcgaacgcct ctgcaagacc acaacagtga ggacaaggta cactgtcatg     300

tccaaggagc tcctggatga acacccagaa ctaaagacag agggcactcc aacattgaca     360

ccacgtcttg acatctgcaa tgctgcggtg cttgaacttg gtgctgctgc agctcgcgcc     420

gcccttggtg aatggggccg tccagcagct catattaccc acctagtcta catctcatcc     480

agcgagcttc gcctcccagg gggggatctt ttcctggcaa cccgtcttgg cctctctcca     540

aacacagtcc gcacatccct tctcttcctc ggctgctcag gtggtgctgc cgccctccgc     600

actgccaagg acattgcaga gaacaaccca gggagccgtg tccttgtaat agctgcggag     660

acaactgtgt tgggcttccg gccaccaagc gctgatcgtc cttatgacct tgttggtgct     720

gctctgtttg gtgatggtgc atcagctgca attattggag caagccctat cagtgcagaa     780

gaggatccct tcctggagct tgagttctcg acccaagagt tcctaccagg gacagacaag     840

gtaattgatg gcaagatctc agaggaaggg atcaacttca aactggggcg tgatctgccg     900

gaaaagattg aaagccgcat agaagggttc tgcaggatac ttatggacaa ggttgggata     960

aagaagttca atgatgtttt ctgggcagtg catcccggtg gaccagcaat attaaacagg    1020

ctggaggttt gtcttgaact tgatccagat agactcaaga tcagtagaaa ggccctgatg    1080

aactatggga atgtgagcag caacacagtc ttctatgtgc tggagtattt gagggatgaa    1140

ttgaagaaag ggacaataag ggaagagtgg ggattaatct tggcttttgg cccaggcatc    1200

acatttgaag ggatgctagt tcggggcatt aactgaaact ggaagggatc caagtaggat    1260

actaccagat acatggaaag atcacaaaca agctttacat ggcacaatac catttcgcaa    1320

ttataattat atagtgtaag aatagcagtt cgcgattata attatatact ataagaatat    1380

cataagaagc atagcgtctt gatgatttct gttcactact tactcagtac tttgttgtat    1440

tttatgcttt atgctatcaa ttatcaaaca atgtcagttg ctcgagtttg ttccca        1496

<210>  3
<211>  1692
<212>  DNA
<213>  Brassica napus

<400>  3
aatcttcacc aaatatcaac taggcagaaa catcgcacaa agatgcaggt catcgactgt      60

atatatatat atatgccttc accttgaccc ttttgaacat actcaatatt taacaagaga     120

aaacagatag aaaatgggaa gcatcgacgc aacagagttg ggttcagaga agaaaccaaa 180

tcctgggaaa gccaccattc ttgctatcgg aaaagccttt cctcaccagc ttgtgatgca 240

agagtatttg gttgatggct acttcaaaac caccaactgt gatgaccctg aactcaaaca 300

gaagcttact cgtctctgta atctctccac tttaaccttc tctctttaaa actttgtata 360

agcttctgaa aactgaggtt gtttgactgt ttcgactact atataggcaa gacaacgacg 420

gtgaagacaa ggtatgttgt aatgtcagag gagatactaa cgaagtatcc agaactcgcc 480

attgaaggag gatccaccgt gaagcagcgt ctggacatat gcaacgacgc tgtcactgaa 540

atggcagtgg aagcttctag agcctgcatc aagaaatggg gccgttctat ctccgacata 600

actcaccttg tctacgtctc ctcaagcgaa gctcgtcttc ctggtggcga cctgtactta 660

gccaaaggac ttggtctcag ccccgagaca caccgtgttc tgctctactt cgtaggctgt 720

tctggtggcg ttgcaggcct ccgtgtagcg aaagacatag ccgagaacaa tccggggagt 780

agagttttgc ttgctacttc tgagacaacc atcattggtt tcaaacctcc aagtgcagac 840

agaccgtatg atcttgttgg ggtcgcgttg tttggtgatg gagctggagc tatgatcatc 900

ggatccgatc cagaccccat ctctgagaag cctctctttg agcttcacac tgcaattcaa 960

aacttccttc ctggtaaaaa aaaactacat gatctttgca tccttataac ttagcctatg 1020

tgtttcgaca aactttagga tagcattttc atggtgcaga cactgagaag accatcgatg 1080

ggaggctaac ggaagaaggg ataaacttca cgctgtcgag agagcttccg cagattatag 1140

aagacaacgt ggagagtttc tgcaagaagc taataggaaa agcgggactg actcctaaag 1200

actataacca gatgttctgg gcggttcatc caggcggacc agccatcttg aaccgaatgg 1260

aaaagcggct taacctctcg ccggagaagc tgagtccaag cagaagagct ctcatggact 1320

acggcaacgc aagtagcaac tccatcgttt atgtgttgga gtatatgttg gaagagagcc 1380

ggaaagcaag gaacatgaag gaagttgaaa acgaatgggg tctgattcta gcttttggac 1440

ctggtgttac atttgaaggc atcgttgctc gaaaccttga tgtttgagca acagagagag 1500

atatgcatgt aataagatgg aagctttaaa gagatgaact cttttttacg atacttaaat 1560

gtttcgtagt ctaaaaatat ttcacaaagc aaaacaaacg tagaatgtgc aatttgaaaa 1620

acaacgaaag agttgttatc agcattaaaa ccagggatca tgaagtttct tctaagctca 1680

tctcttgcac ac 1692

<210> 4
<211> 1535
<212> DNA
<213> Brassica napus

<400> 4
ctaatcttca ccaaatatca actagacaga acacaccgca caagacgcag gtcataaact 60

gcaaaagcgt tagtgtatat atatatatat ctttactttg actctttatt acagacacaa 120

caagatttga gtcttttcac aagagcaaca agattgtaat ggggagcatc gatgccacag 180

agttgggttc agagaagaaa ccaaaccctg ggaaagcgac cattcttgct atcggaaaag 240

cctttcctca ccagcttgtg atgcaagagt acttggttga tggctacttc aaaaccacca 300

actgtgatga ccctgaactc aaacagaagc ttactcgcct ctgcaagaca accacggtga 360

agacaaggta cgttgtaatg tcagaggaga tactaacaaa gtatccagaa ctcgccatcg 420

aaggaggatc caccgtgaag cagcgtctgg acatatgcaa cgacgctgtc accgaaatgg 480

cggtagaagc ttctagagcc tgcatcaaga aatggggccg ttctatctcc gacataactc 540

accttgtcta cgtctcctca agcgaagctc gtcttcctgg tggcgacctg tacttagcca 600

aaggacttgg tctcagcccc gagacacacc ccgttcttct ctacttcgta ggctgttctg 660

gtggcgttgc aggcctccgt gtagccaaag acatagccga gaacaatccg gggagtagag 720

ttttgcttgc tacttctgag actaccatca ttggtttcaa acctccaagt gcagacagac 780

cgtatgatct tgttggggtc gcgttgtttg gtgatggagc tggagctatg atcatcggat 840

ccgacccaga ccccatctct gagaagcctc tctttgagct tcacactgca attcaaaatt 900

tccttcctgg taaaaaacta catgatcttt gcatccttat aacttagccg atgtgtttag 960

acaaacttag gatagcattt tcatggtgca gacacagaga agacgataga tgggaggcta 1020

acggaagaag ggataaactt cacgctgtcg agagagcttc cgaagctaat agaagacaac 1080

gtggagagtt tctgcaagaa gctaatagga aaagcgggac tgactcctaa agactataac 1140

cagatgttct gggcggttca tccaggcgga ccagccatct tgaaccgaat ggaaaagcgg 1200

cttaacctct caccggagaa gctgagtcca agcaggagag ctctcatgga ctacggcaac 1260

gcaagtagca actcgatcgt ttatgtgttg gagtatatgt tggaagagag ccggaaagca 1320

aggaacatga aggaagttga aaacgaatgg ggtctgattc tagcttttgg acctggtgtt 1380

acatttgaag gcatcgttgc tcgaaacctt gatgtttgag caacagagag agatatgcat 1440

gtaataagat ggaagcttta aagagatgaa ctctttttta cgatacttaa atgtttcgta 1500

gtctaaaaat atttcacaaa gcaaaacaaa cgtag 1535

<210> 5
<211> 1801
<212> DNA
<213> Brassica napus

<400> 5
ctaatcttca ccaaatatca actagacaga acacaccgca caagacgcag gtcataaact 60

gcaaaagcgt tagtgtatat atatatatat ctttactttg actctttatt acagacacaa 120

caagatttga gtcttttcac aagagcaaca agattgtaat ggggagcatc gatgccacag 180

agttgggttc agagaagaaa ccaaaccctg ggaaagcgac cattcttgct atcggaaaag 240

cctttcctca ccagctagtg atgcaagagt acttggtcga tggctacttc aaaaccacaa 300

actgtgacga ccctgaactc aaacagaagc ttactcgcct ctgcaagaca accacggtga 360

agacaaggta cgttgtaatg tcagaggaga tactaacaaa gtatccagaa ctcgccatcg 420

aaggaggatc caccgtgaag cagcgtctgg acatatgcaa cgacgctgta accgaaatgg 480

cagtggaagc ttcaagatcc tgcatcaaaa actggggccg ttctgtttcc gacataactc 540
accttgttta tgtctcctca agcgaagctc gtcttcctgg aggcgacttg ttcttagcca 600
aagggcttgg tctcagcccc gagacgcacc gggttctgct ctacttcgtc ggctgctctg 660
gaggcgttgc tggtctccgt gtagccaaag acatagccga gaacaatccg gggagtagag 720
tcttgcttgc cacgtcagag acgaccatca ttggtttcaa accgccgagt aaagatagac 780
cgtacgatct tgttggtgtc gcgttgtttg gtgatggagc cggagctatg atcatcgggt 840
ctgatccaga cccggttagt gagaagccac tctttgagct tcacaccgca attcaaaatt 900
ttctacctgg taaaaaacta caagactttt gcatccaaca atgcctataa ccagaaccgt 960
tctagacaca atcggatgaa acatttactt gagaaatttt ttcataaata cttttttttt 1020
ggtcaaatat tttcataaat actttaagat atatttaaaa attatttata aaacacttat 1080
ataagtttat aaacccaatt ctacttccaa aatactaaac cctaaacaat aatcacattt 1140
aaactcaaat attttgtttt tttattgagt atatttttga aaagttggtg gtcactttag 1200
ggtattttaa acaatttctc aaaaaaaaaa ttatgatcca ataaatctta gatctggatt 1260
aactttcgtg tcgcagacac ggagaagaca atagatggga ggctaacgga atcaggcata 1320
aacttcacac tatcaagaga gcttccacag ataatagaag acaacgtgga gagtttctgc 1380
aagaagctac taggaaaagc aggactggct cataaagact ataaccagat gttttgggcg 1440
gttcatccag gtggaccagc catattgaac cgaatggaaa agcggcttaa cctgtcgccg 1500
gagaagctga gtccaagcag aagatctctc atggactatg gcaacgcaag tagcaattcg 1560
atcgtttacg tgttggagta tatgttggag gagagcagga aagcaaggaa gatgaatgaa 1620
ggtgaaaatg agtggggtct gattctggct tttggacctg gtgttacatt tgaaggtatc 1680
gttgcaagga atctcgatgt ttgagcagaa agtgggtgtt ttggttctac cttgaggctc 1740
aagaaacagt ccccttttgc tttatagtca tttcaacaga gagagaaatg catataataa 1800
g 1801

## Description

### Field of Art

The present invention relates to a method of production of hybrid F1 seed of angiosperm plants.

### Background Art

The growing global population and increasing food consumption place increased demands on the productivity of plant production. The breeding progress in the important agricultural crops such as wheat, barley and rape achieved using current technology is slowing down. A possible solution to this problem is the use of heterotic effect - breeding hybrid varieties and producing hybrid seeds. At present, there are several methods of producing hybrid seeds, but they all have serious drawbacks:
Cytoplasmic sterility (CMS) - was described in extra-variety cross-breeding such as in the *Triticum timopheevii x Triticum aestivum* (wheat), *Hordeum spontaneum x Hordeum vulgare* (barley) and *Raphanus raphanistrum x Brassica napus* (rape). Its use is problematic due to the demanding and long-lasting conversion of cytoplasm to a cultivated genome. This process lasts a number of years. However, it's absolutely essential, both for reasons of agronomic performance and because of the necessity to have available a sister line which maintains sterility, and which has the same genome and differs by its cytoplasm, which is fertile. At present, CMS is widely used with rape, but its use with barley is marginal. It's not used with wheat, due to the negative effect of the *Triticum timopheevii* cytoplasm; a fundamental drawback is the unavailability of fertility restorers (Cisar and Cooper, 2002).

Chemically induced sterility (CHA) - at present, this is the most widely used system of producing hybrid seed in wheat; its use, however, is risky, costly and inefficient from a seed-growing perspective. The riskiness is largely due to sensitivity in the application phase and environmental conditions at the time of application, the costliness is due to the need to use relatively large doses of expensive chemicals, and the low seed-growing productivity due to the need to grow the maternal and paternal components in adjacent strips, resulting in a 50% reduction in yield per unit of area. This method can also be used with barley and rape, but the same problems occur for barley and rape as for wheat. Genetic modification (GMO) - several systems exist which are based on the use of genetic modifications of the nuclear genome, which generally have a lowered seed-growing productivity in the maintenance of a sterile maternal component - for example in the system of producing a hybrid wheat seed described in EP2294204B1, it is essential in this phase to use herbicide to kill 50% of the area of vegetation where the maintenance of the sterile maternal component takes place. Furthermore, due to the conditions of EU legislation and the antagonism of the population towards GMO, these systems do not have any practical applicability.

### Disclosure of the Invention

The present invention provides a method of producing a hybrid F1 seed of an angiosperm plant. Within the framework of this invention, it has been found that the inactivation of a gene involved in the synthesis of sporopollenin results in complete sterility of a plant, and that this sterility may be artificially reversed to a fertile state by spraying with 4-oxo-6-octadecane-pyran-2-olate.

The method of producing a hybrid F1 seed of an angiosperm plant according to the invention contains the following steps:
- a maternal line is prepared by inactivation of a gene involved in the synthesis of sporopollenin. The genes involved in the synthesis of sporopollenin include genes for the synthesis of sporopollenin or of a sporopollenin precursor. The inactivation may be carried out by chemical mutagenesis or any other suitable gene inactivation method. The inactivation of the gene results in induction of pollen sterility. At the same time, mutation of acetolactate synthase gene is carried out, resulting in induction of tolerance to herbicides of the acetolactate synthase inhibitor group;
- a paternal line is prepared, which is a fertile line without resistance to herbicides of the acetolactate synthase inhibitor group;
- subsequently, hybrid F1 seeds are produced by mixing the seed of the sterile herbicide-tolerant maternal line and the fertile herbicide-sensitive paternal line in a 95:5 ratio and by subsequent sowing this mixture of seed of the maternal line and paternal line to produce the desired hybrid F1 seeds; and

- after the flowering period of the vegetation of mixture of the maternal line and paternal line, the plants are treated with a herbicide of the acetolactate synthase inhibitor group, thereby killing the herbicide-sensitive paternal line plants and leaving the herbicide-resistant maternal line plants producing pure fertile hybrid F1 seed;
- whereas the sterile maternal line is maintained by spraying with 4-oxo-6-octadecane-pyran-2-olate, preferably in a dose of 2-5 kg.ha⁻¹, in the phase in which pollen tetrads are formed.

Problems of the current state of the art are thus solved by the method of artificial induction of genomic sterility and its use for the preparation of hybrid F1 seed of an angiosperm plant. The method includes the steps of production of starting materials, which in a preferred embodiment are prepared as follows:
- The maternal line: The nuclear genome of the maternal line carries inactivated gene(s) involved in the synthesis of sporopollenin, which results in pollen sterility. The inactivation is preferably carried out by a chemical mutagen, more preferably by ethyl methanesulfonate. The nuclear genome of the maternal line further carries a mutated acetolactate synthase gene which provides tolerance to herbicides from the acetolactate synthase inhibitor group. The mutation is preferably carried out by a chemical mutagen, more preferably by ethyl methanesulfonate.

In a preferred embodiment, the maternal line can be prepared by crossing a first line with inactivated at least one gene involved in the synthesis of sporopollenin and a second line with a mutated acetolactate synthase gene.
- The paternal line: The paternal line is a fertile line without resistance to herbicide from the acetolactate synthase inhibitor group.

The step of production of the hybrid F1 seed from the starting maternal line and paternal line includes:
- The maintenance of the maternal line, for the purpose of its multiplication, which is performed by spraying with 4-oxo-6-octadecane-pyran-2-olate, preferably in a dose of 2-5 kg.ha⁻¹, in the phase in which pollen tetrads are formed.
- The production of the hybrid F1 seed: seed of the maternal line are mixed with seed of the paternal line in a ratio of 95:5 (maternal:paternal) and sown. During the flowering period, the sterile maternal line is pollinated by the fertile paternal line. After the flowering period ends, all plants are treated with herbicide from the acetolactate synthase inhibitor group. The herbicide-sensitive paternal line plants are killed by this treatment, and the maternal line plants remain in the vegetation, producing the pure fertile hybrid F1 seed.

In one embodiment of the invention, the angiosperm plant is selected from wheat, barley and rape.

The genes involved in the synthesis of sporopollenine are preferably selected from TaSpPS and its homologs in wheat, HvSpPS and/or its homologs in barley, BnSpPS1, BnSpPS2, BnSpPS3 and their homologs in rape, or orthologs and/or homologs of these genes in other angiosperm plants.

The term "maternal line" designates breeding line, plants and seed carrying in the nuclear genome inactivated gene(s) involved in the synthesis of sporopollenin and a mutated acetolactate synthase gene.

The term "paternal line" designates breeding line, plants and seed which is/are fertile and do not show resistance to herbicide from the acetolactate synthase inhibitor group.

### Examples of carrying out the invention

### Example 1: Preparation of wheat hybrid F1 seed

TaSpPS is a wheat gene for synthesizing a sporopollenin precursor having the following sequence (SEQ. ID.NO.1):

CATGGCGAGCATCCCCTATTGAAGCGAAGCACCCATCACGCACACAGTGAAAT
CACAGGAGCGGAGAACTTGTAAACCTGAAGCGGAAGCAAGCAAGGAGCGCCT
GTGAGGCAGCGATGTCTGGCCTTGGTGGACTCGGGAGCATCGGCGGGGCGGC
AACGGCATAAGCCACGGCTCGGGGGCAAGGCCAAGCTGCTCGCACTCGGCA
AGGGTCTCCCTGAACAAGTTCTACCCCAGGAGAAGCTCGTCGAGACCTACCTC
CAGGACACCAGCTGCGACGATCCCGCCACCAGGGCCAAGCTCGAACGCCTCTG

CAAGACCACAACAGTGAGGACAAGGTACACTGTCATGTCGAAGGAGCTCCTG
GATGAATACCCAGAATTGAAGACAGAAGGCTCTCCAACATTGACGCCACGGCT
TGACATCTGCAATGCCGCGGTGCTTGAACTTGGTGCTGCTGCAGCTCGTGCCGC
CCTTGGTGAGTGGGGCCGTCCAGCAGCTGATATTACCCACCTGGTCTACATCTC
CTCCAGCGAGCTTCGCCTCCCAGGGGGTGATCTTTTCCTGGCAACCCGTCTTGG
CCTCTCTCCAAACACAGTGCGCACATCCCTTCTCTTCCTTGGCTGCTCAGGTGG
TGCTGCCGCCCTCCGCACTGCCAAGGACATTGCAGAGAACAACCCAGGGAGCC
GTGTCCTTGTAATAGCGGCGGAGACAACTGTGTTGGGCTTCCGGCCGCCAAGC
CCTGATCGTCCTTATGACCTCGTTGGTGCTGCTCTGTTTGGTGATGGTGCATCA
GCTGCAATTATTGGAGCAAGCCCTATCAAAGCAGAAGAGGATCCTTTCTTGGA
GCTTGAGTTCTCAACCCAGGAGTTCCTACCAGGGACAGACAAGGTAATTGATG
GCAAGATCTCAGAGGAAGGGATTAACTTCAAACTGGGGCGTGATTTGCCTGAA
AAGATTGAAAGCCGCATAGAAGGCTTCTGCAGGATACTCATGGACAAGGTTGG
GATAAAGGAGTTCAATGACGTATTTTGGGCTGTGCATCCCGGCGGACCAGCAA
TATTGAACAGGCTGGAGGTTTGTCTTGAACTTGAGCCAGATAAACTCAAGATC
AGTAGAAAGGCCCTGATGAACTATGGGAATGTGAGCAGCAACACAGTCTTCTA
TGTGCTGGAGTATTTGAGGGATGAATTGAAGAAAGGGGCAATAAGGAAGAG
TGGGGATTGATCTTGGCTTTTGGCCCAGGCATCACATTTGAAGGAATGCTAGTT
CGGGGCATTAACTGAAATTGAAGGAGATCCAAGTAGGTTACTACCAGATACAT
GGAAAGATCAAAAACAAGCTTTATATGGCACAGTAGCATTTCACAATTATAAT
TATACAGTGTAAGGATAGCAATTCGCGGCTATAATTATATACTGTAAGAATAT
CATAAGCATAGCGTCTTGATGATTTCTGTTCACTACATACTCAGCAC

The inactivation of TaSpPS and its homologues by chemical mutagenesis, and the identification of the lines carrying the eliminated TaSpPS genes, was performed using standard TILLING technology described in literature (Uauy et al. 2009). In wheat, all three homologues of the TaSpPS gene must be inactivated. The lines carrying the inactivated genes in their individual genomes are identified, and then by their crossing and selection the lines carrying the inactivated TaSpPS gene and its homologs were prepared.

Induction of the resistance of wheat to herbicides from the acetolactate synthase inhibitor group was carried out as described in the literature (EP0508161).

The maternal wheat line was prepared by crossing the lines carrying the inactivated TaSpPS gene and its homologs and the lines resistant to herbicides from the acetolactate synthase inhibitor group. In the F2 progeny, the selection of genotypes carrying resistance to herbicides from the acetolactate synthase inhibitor group was performed by spraying them with a herbicide from this group. In the surviving plants, identification of genotypes carrying inactivated TaSpPS and its homologs was performed.

The maternal wheat line was maintained by spraying with 4-oxo-6-octadecane-pyran-2-olate in a dose of 2-5 kg.ha⁻¹ in the form of water emulsion in the phase in which pollen tetrads are formed. The biological effectiveness of 4-oxo-6-octadecane-pyran-2-olate is tested by protecting parts of the plants with an insulating bag before spraying. The insulating bag is then applied to the spike during flowering. As a control variant, identical procedure is performed with plants in which homologues of the TaSpPS have not been inactivated (the Bohemia variety of wheat). The degree of sterility and the degree of restoration of fertility is expressed by the percent of grains created by the maternal line (TaSt06) compared to the untreated control variant (Bohemia). The degree of potential toxicity is expressed by the reduction of the number of grains in the treated (sprayed) control variant compared to the untreated (insulated) control variant, expressed in %. The summarized results of the testing are shown in the following table:

| | Number of grains per spike | | | | | |
|---|---|---|---|---|---|---|
| Dose of oxo-6-octadecane-pyran-2-olate | 2 kg.ha⁻¹ | | 3.5 kg.ha⁻¹ | | 5 kg.ha⁻¹ | |
| Line | Insulation | Spraying | Insulation | Spraying | Insulation | Spraying |
| Bohemia | 36 | 36 | 38 | 37 | 37 | 35 |
| TaSt06 | 0 | 30 | 0 | 35 | 0 | 35 |
| Sterility (%) | 100 | | 100 | | 100 | |
| Restoration of fertility (%) | 83 | | 92 | | 95 | |
| Toxicity (%) | 0 | | 3 | | 5 | |

The inactivation of TaSpPS and of the homologues of the TaSpPS gene, therefore, resulted in an induction of 100% sterility; treatment with oxo-6-octadecane-pyran-2-olate resulted in an 83-95% restoration of fertility, whereby toxicity manifested itself at a level of 0-5%.

Production of F1 hybrid wheat seed: the seed from the maternal line is mixed with the seed from the paternal line in a ratio of 95:5, and sown. At the time of flowering, the sterile maternal line is pollinated by the fertile paternal line. After the flowering ends, the vegetation is treated with herbicide from the acetolactate synthase inhibitor group. The herbicide-sensitive paternal line is killed, and the maternal line which produces the pure fertile hybrid F1 seed remains in the vegetation.

The testing of the heterotic effect of the created F1 wheat seed took place using standard experimental methodology. The F1 seed was, together with control varieties (marked *), sown in micro-parcels in two repetitions and in two localities. The harvested grain was weighed, and the results were statistically analysed. The summarized results of the testing are shown in the following table:

| | Yield % | | |
|---|---|---|---|
| Localities: | Úh 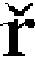 etice | Stupice | Average |
| F1 Bohemia x TaSt06 | 126 | 128 | 127 |
| Bohemia* | 105 | 100 | 102.5 |
| Turandot* | 103 | 102 | 102.5 |

### Example 2: Preparation of barley hybrid F1 seed

HvSpPS is a barley gene for synthesizing a sporopollenin precursor having the following sequence (SEQ. ID.NO.2):

GAGCGAAATCACAGGAGCGGAGAAATTCTAAACCTGAGGCAGAAGGAAGCAA
CGAGCGTCTGTGAGTCAGCGATGTCTGGCCTTGGAAGCATCGGCGGGGCGGC
AACGGCAGAAGCCATGGTTCAGGGGGCAAGGCCAAGCTGCTCGCGCTCGGCA
AGGGTCTCCCTGAGCAAGTTCTACCCCAGGAGAAGCTCGTCGAGACCTACCTC
CAGGACACCAGCTGCGACGATCCCGCCACCAGGGCCAAGCTCGAACGCCTCTG
CAAGACCACAACAGTGAGGACAAGGTACACTGTCATGTCCAAGGAGCTCCTGG
ATGAACACCCAGAACTAAAGACAGAGGGCACTCCAACATTGACACCACGTCTT
GACATCTGCAATGCTGCGGTGCTTGAACTTGGTGCTGCTGCAGCTCGCGCCGCC
CTTGGTGAATGGGGCCGTCCAGCAGCTCATATTACCCACCTAGTCTACATCTCA

TCCAGCGAGCTTCGCCTCCCAGGGGGGATCTTTTCCTGGCAACCCGTCTTGGC
CTCTCTCCAAACACAGTCCGCACATCCCTTCTCTTCCTCGGCTGCTCAGGTGGT
GCTGCCGCCCTCCGCACTGCCAAGGACATTGCAGAGAACAACCCAGGGAGCC
GTGTCCTTGTAATAGCTGCGGAGACAACTGTGTTGGGCTTCCGGCCACCAAGC
GCTGATCGTCCTTATGACCTTGTTGGTGCTGCTCTGTTTGGTGATGGTGCATCA
GCTGCAATTATTGGAGCAAGCCCTATCAGTGCAGAAGAGGATCCCTTCCTGGA
GCTTGAGTTCTCGACCCAAGAGTTCCTACCAGGGACAGACAAGGTAATTGATG
GCAAGATCTCAGAGGAAGGGATCAACTTCAAACTGGGGCGTGATCTGCCGGA
AAAGATTGAAAGCCGCATAGAAGGGTTCTGCAGGATACTTATGGACAAGGTTG
GGATAAAGAAGTTCAATGATGTTTTCTGGGCAGTGCATCCCGGTGGACCAGCA
ATATTAAACAGGCTGGAGGTTTGTCTTGAACTTGATCCAGATAGACTCAAGAT
CAGTAGAAAGGCCCTGATGAACTATGGGAATGTGAGCAGCAACACAGTCTTCT
ATGTGCTGGAGTATTTGAGGGATGAATTGAAGAAAGGACAATAAGGAAGA
GTGGGGATTAATCTTGGCTTTTGGCCCAGGCATCACATTTGAAGGATGCTAGT
TCGGGGCATTAACTGAAACTGGAAGGGATCCAAGTAGGATACTACCAGATACA
TGGAAAGATCACAAACAAGCTTTACATGGCACAATACCATTTCGCAATTATAA
TTATATAGTGTAAGAATAGCAGTTCGCGATTATAATTATATACTATAAGAATAT
CATAAGAAGCATAGCGTCTTGATGATTTCTGTTCACTACTTACTCAGTACTTTG
TTGTATTTTATGCTTTATGCTATCAATTATCAAACAATGTCAGTTGCTCGAGTTT
GTTCCCA

The inactivation of HvSpPS gene in barley by chemical mutagenesis, and the identification of lines carrying the eliminated HvSpPS gene, was carried out using the standard TILLING technology described in the literature (Bovina et al. 2011).

Induction of the resistance of barley to herbicides from the acetolactate synthase inhibitor group was carried out as described in the literature Lee et al. (2011).

The maternal barley line is prepared by crossing the lines carrying the inactivated HvSpPS gene and the lines resistant to acetolactate synthase inhibitor herbicides. In the F2 progeny, the selection of genotypes carrying resistance to herbicides from the acetolactate synthase inhibitor group is carried out by spraying them with a herbicide from this group. In the surviving plants, identification of genotypes carrying the eliminated gene HvSpPS is performed.

The maternal barley line is maintained by spraying with 4-oxo-6-octadecane-pyran-2-olate in a dose of 2-5 kg.ha⁻¹ in the form of water emulsion in the phase in which pollen tetrads are formed. The biological effectiveness of 4-oxo-6-octadecane-pyran-2-olate is tested by protecting parts of the plants with an insulating bag before spraying. The insulating bag is then applied to the spike during flowering. As a control variant, an identical procedure is performed with plants in which HvSpPS have not been inactivated (the barley variety Lancelot). The degree of sterility and the degree of restoration of fertility is expressed by the percent of grains created by the maternal line (HvSt03) compared to the untreated control variant (Lancelot). The degree of potential toxicity is expressed by the reduction of the number of grains in the treated (sprayed) control variant compared to the untreated (insulated) control variant, expressed in %. The summarized results of the testing are shown in the following table:

| | Number of grains per spike | | | | | |
|---|---|---|---|---|---|---|
| Dose of oxo-6-octadecane-pyran-2-olate | 2 kg.ha⁻¹ | | 3.5 kg.ha⁻¹ | | 5 kg.ha⁻¹ | |
| Line | Insulation | Spraying | Insulation | Spraying | Insulation | Spraying |
| Lancelot | 35 | 35 | 35 | 34 | 35 | 33 |
| HvSt03 | 0 | 30 | 0 | 34 | 0 | 33 |
| Sterility (%) | 100 | | 100 | | 100 | |
| Restoration of fertility (%) | 86 | | 97 | | 94 | |
| Toxicity (%) | 0 | | 3 | | 6 | |

The inactivation of the HvSpPS gene, therefore, resulted in the induction of 100% sterility; treatment with oxo-6-octadecane-pyran-2-olate resulted in an 86-97% restoration of fertility, whereby toxicity manifested itself at a level of 0-6%.

Production of F1 hybrid barley seed: the seed from the maternal line is mixed with the seed from the paternal line in a ratio of 95:5, and sown. At the time of flowering, the sterile maternal line is pollinated by the fertile paternal line. After the flowering ends, the vegetation is treated with herbicide from the acetolactate synthase inhibitor group. The herbicide-sensitive paternal line is killed, and the maternal line which produces the pure fertile hybrid F1 seed stays in the vegetation.

The testing of the heterotic effect of the created F1 barley seed took place using standard experimental methodology. The F1 seed was, together with control varieties (marked *), sown in micro-parcels in two repetitions and in two localities. The harvested grain was weighed, and the results were statistically analysed. The summarized results of the testing are shown in the following table:

| | Yield % | | |
|---|---|---|---|
| | Lu 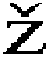 any | Stupice | Average |
| F1 Lancelot x TaSt03 | 118 | 121 | 119.5 |
| Lancelot* | 98 | 99 | 98.5 |
| Sylva* | 101 | 102 | 101.5 |

### Example 3: Preparation of rape hybrid F1 seed

BnSpPS1 is a rape gene for synthesizing a sporopollenin precursor having the following sequence (SEQ. ID.NO.3):

AATCTTCACCAAATATCAACTAGGCAGAAACATCGCACAAAGATGCAGGTCAT
CGACTGTATATATATATATATGCCTTCACCTTGACCCTTTTGAACATACTCAAT
ATTTAACAAGAGAAAACAGATAGAAAATGGGAAGCATCGACGCAACAGAGTT
GGGTTCAGAGAAGAAACCAAATCCTGGGAAAGCCACCATTCTTGCTATCGGAA
AAGCCTTTCCTCACCAGCTTGTGATGCAAGAGTATTTGGTTGATGGCTACTTCA
AAACCACCAACTGTGATGACCCTGAACTCAAACAGAAGCTTACTCGTCTCTGT
AATCTCTCCACTTTAACCTTCTCTCTTTAAAACTTTGTATAAGCTTCTGAAAACT
GAGGTTGTTTGACTGTTTCGACTACTATATAGGCAAGACAACGACGGTGAAGA
CAAGGTATGTTGTAATGTCAGAGGAGATACTAACGAAGTATCCAGAACTCGCC
ATTGAAGGAGGATCCACCGTGAAGCAGCGTCTGGACATATGCAACGACGCTGT
CACTGAAATGGCAGTGGAAGCTTCTAGAGCCTGCATCAAGAAATGGGGCCGTT
CTATCTCCGACATAACTCACCTTGTCTACGTCTCCTCAAGCGAAGCTCGTCTTC
CTGGTGGCGACCTGTACTTAGCCAAAGGACTTGGTCTCAGCCCCGAGACACAC
CGTGTTCTGCTCTACTTCGTAGGCTGTTCTGGTGGCGTTGCAGGCCTCCGTGTA

GCGAAAGACATAGCCGAGAACAATCCGGGGAGTAGAGTTTTGCTTGCTACTTC
TGAGACAACCATCATTGGTTTCAAACCTCCAAGTGCAGACAGACCGTATGATC
TTGTTGGGGTCGCGTTGTTTGGTGATGGAGCTGGAGCTATGATCATCGGATCCG
ATCCAGACCCCATCTCTGAGAAGCCTCTCTTTGAGCTTCACACTGCAATTCAAA
ACTTCCTTCCTGGTAAAAAAAAACTACATGATCTTTGCATCCTTATAACTTAGC
CTATGTGTTTCGACAAACTTTAGGATAGCATTTTCATGGTGCAGACACTGAGAA
GACCATCGATGGGAGGCTAACGGAAGAAGGATAAACTTCACGCTGTCGAGA
GAGCTTCCGCAGATTATAGAAGACAACGTGGAGAGTTTCTGCAAGAAGCTAAT
AGGAAAAGCGGGACTGACTCCTAAAGACTATAACCAGATGTTCTGGGCGGTTC
ATCCAGGCGGACCAGCCATCTTGAACCGAATGGAAAAGCGGCTTAACCTCTCG
CCGGAGAAGCTGAGTCCAAGCAGAAGAGCTCTCATGGACTACGGCAACGCAA
GTAGCAACTCCATCGTTTATGTGTTGGAGTATATGTTGGAAGAGAGCCGGAAA
GCAAGGAACATGAAGGAAGTTGAAAACGAATGGGGTCTGATTCTAGCTTTTGG
ACCTGGTGTTACATTTGAAGGCATCGTTGCTCGAAACCTTGATGTTTGAGCAAC
AGAGAGAGATATGCATGTAATAAGATGGAAGCTTTAAAGAGATGAACTCTTTT
TTACGATACTTAAATGTTTCGTAGTCTAAAAATATTTCACAAAGCAAAACAAA
CGTAGAATGTGCAATTTGAAAAACAACGAAAGAGTTGTTATCAGCATTAAAAC
CAGGGATCATGAAGTTTCTTCTAAGCTCATCTCTTGCACAC

BnSpPS2 is a rape gene for synthesizing a sporopollenin precursor having the following sequence (SEQ. ID.NO.4):

CTAATCTTCACCAAATATCAACTAGACAGAACACACCGCACAAGACGCAGGTC
ATAAACTGCAAAAGCGTTAGTGTATATATATATATCTTTACTTTGACTCTTT
ATTACAGACACAACAAGATTTGAGTCTTTTCACAAGAGCAACAAGATTGTAAT
GGGGAGCATCGATGCCACAGAGTTGGGTTCAGAGAAGAAACCAAACCCTGGG
AAAGCGACCATTCTTGCTATCGGAAAAGCCTTTCCTCACCAGCTTGTGATGCAA
GAGTACTTGGTTGATGGCTACTTCAAAACCACCAACTGTGATGACCCTGAACT
CAAACAGAAGCTTACTCGCCTCTGCAAGACAACCACGGTGAAGACAAGGTAC
GTTGTAATGTCAGAGGAGATACTAACAAAGTATCCAGAACTCGCCATCGAAGG
AGGATCCACCGTGAAGCAGCGTCTGGACATATGCAACGACGCTGTCACCGAAA
TGGCGGTAGAAGCTTCTAGAGCCTGCATCAAGAAATGGGGCCGTTCTATCTCC
GACATAACTCACCTTGTCTACGTCTCCTCAAGCGAAGCTCGTCTTCCTGGTGGC
GACCTGTACTTAGCCAAAGGACTTGGTCTCAGCCCCGAGACACACCCCGTTCT

TCTCTACTTCGTAGGCTGTTCTGGTGGCGTTGCAGGCCTCCGTGTAGCCAAAGA
CATAGCCGAGAACAATCCGGGGAGTAGAGTTTTGCTTGCTACTTCTGAGACTA
CCATCATTGGTTTCAAACCTCCAAGTGCAGACAGACCGTATGATCTTGTTGGGG
TCGCGTTGTTTGGTGATGGAGCTGGAGCTATGATCATCGGATCCGACCCAGAC
CCCATCTCTGAGAAGCCTCTCTTTGAGCTTCACACTGCAATTCAAAATTTCCTT
CCTGGTAAAAAACTACATGATCTTTGCATCCTTATAACTTAGCCGATGTGTTTA
GACAAACTTAGGATAGCATTTTCATGGTGCAGACACAGAGAAGACGATAGATG
GGAGGCTAACGGAAGAAGGGATAAACTTCACGCTGTCGAGAGAGCTTCCGAA
GCTAATAGAAGACAACGTGGAGAGTTTCTGCAAGAAGCTAATAGGAAAAGCG
GGACTGACTCCTAAAGACTATAACCAGATGTTCTGGGCGGTTCATCCAGGCGG
ACCAGCCATCTTGAACCGAATGGAAAAGCGGCTTAACCTCTCACCGGAGAAGC
TGAGTCCAAGCAGGAGAGCTCTCATGGACTACGGCAACGCAAGTAGCAACTCG
ATCGTTTATGTGTTGGAGTATATGTTGGAAGAGAGCCGGAAAGCAAGGAACAT
GAAGGAAGTTGAAAACGAATGGGGTCTGATTCTAGCTTTTGGACCTGGTGTTA
CATTTGAAGGCATCGTTGCTCGAAACCTTGATGTTTGAGCAACAGAGAGAGAT
ATGCATGTAATAAGATGGAAGCTTTAAAGAGATGAACTCTTTTTACGATACTT
AAATGTTTCGTAGTCTAAAAATATTTCACAAAGCAAAACAAACGTAG

BnSpPS3 is a rape gene for synthesizing a sporopollenin precursor having the following sequence (SEQ. ID.NO.5):

CTAATCTTCACCAAATATCAACTAGACAGAACACACCGCACAAGACGCAGGTC
ATAAACTGCAAAAGCGTTAGTGTATATATATATATCTTTACTTTGACTCTTT
ATTACAGACACAACAAGATTTGAGTCTTTTCACAAGAGCAACAAGATTGTAAT
GGGGAGCATCGATGCCACAGAGTTGGGTTCAGAGAAGAAACCAAACCCTGGG
AAAGCGACCATTCTTGCTATCGGAAAAGCCTTTCCTCACCAGCTAGTGATGCA
AGAGTACTTGGTCGATGGCTACTTCAAAACCACAAACTGTGACGACCCTGAAC
TCAAACAGAAGCTTACTCGCCTCTGCAAGACAACCACGGTGAAGACAAGGTAC
GTTGTAATGTCAGAGGAGATACTAACAAAGTATCCAGAACTCGCCATCGAAGG
AGGATCCACCGTGAAGCAGCGTCTGGACATATGCAACGACGCTGTAACCGAAA
TGGCAGTGGAAGCTTCAAGATCCTGCATCAAAAACTGGGGCCGTTCTGTTTCC
GACATAACTCACCTTGTTTATGTCTCCTCAAGCGAAGCTCGTCTTCCTGGAGGC
GACTTGTTCTTAGCCAAAGGGCTTGGTCTCAGCCCCGAGACGCACCGGGTTCT
GCTCTACTTCGTCGGCTGCTCTGGAGGCGTTGCTGGTCTCCGTGTAGCCAAAGA

CATAGCCGAGAACAATCCGGGAGTAGAGTCTTGCTTGCCACGTCAGAGACGA
CCATCATTGGTTTCAAACCGCCGAGTAAAGATAGACCGTACGATCTTGTTGGT
GTCGCGTTGTTTGGTGATGGAGCCGGAGCTATGATCATCGGGTCTGATCCAGA
CCCGGTTAGTGAGAAGCCACTCTTTGAGCTTCACACCGCAATTCAAAATTTCT
ACCTGGTAAAAACTACAAGACTTTTGCATCCAACAATGCCTATAACCAGAAC
CGTTCTAGACACAATCGGATGAAACATTTACTTGAGAAATTTTTCATAAATAC
TTTTTTTTGGTCAAATATTTCATAAATACTTTAAGATATATTTAAAATTATT
TATAAAACACTTATATAAGTTTATAAACCCAATTCTACTTCCAAAATACTAAAC
CCTAAACAATAATCACATTTAAACTCAAATATTTGTTTTTTATTGAGTATATT
TTTGAAAAGTTGGTGGTCACTTTAGGGTATTTTAAACAATTTCTCAAAAAAAAA
ATTATGATCCAATAAATCTTAGATCTGGATTAACTTTCGTGTCGCAGACACGGA
GAAGACAATAGATGGGAGGCTAACGGAATCAGGCATAAACTTCACACTATCA
AGAGAGCTTCCACAGATAATAGAAGACAACGTGGAGAGTTTCTGCAAGAAGC
TACTAGGAAAAGCAGGACTGGCTCATAAAGACTATAACCAGATGTTTTGGGCG
GTTCATCCAGGTGGACCAGCCATATTGAACCGAATGGAAAAGCGGCTTAACCT
GTCGCCGGAGAAGCTGAGTCCAAGCAGAAGATCTCTCATGGACTATGGCAACG
CAAGTAGCAATTCGATCGTTTACGTGTTGGAGTATATGTTGGAGGAGAGCAGG
AAAGCAAGGAAGATGAATGAAGGTGAAAATGAGTGGGGTCTGATTCTGGCTTT
TGGACCTGGTGTTACATTTGAAGGTATCGTTGCAAGGAATCTCGATGTTTGAGC
AGAAAGTGGGTGTTTTGGTTCTACCTTGAGGCTCAAGAAACAGTCCCCTTTTGC
TTTATAGTCATTTCAACAGAGAGAGAAATGCATATAATAAG

The inactivation of the BnSpPS genes by chemical mutagenesis, and the identification of the lines carrying the eliminated BnSpPS genes, was performed with standard TILLING technology described in literature (Gilchrist et al. 2013). In rape, the three orthologous genes BnSpPS1, BnSpPS2 and BnSpPS3 must be inactivated on both genomes. The lines carrying the inactivated genes in their individual genomes are identified, and then by their crossing and selection the lines carrying the inactivated BnSpPS genes were prepared. Induction of the resistance of rape to herbicides from the acetolactate synthase inhibitor group was carried out as described in the literature (Swanston et al., 1989).

The maternal rape line was prepared by crossing the lines carrying the inactivated BnSpPS genes and the lines resistant to herbicides from the acetolactate synthase inhibitor group. In the F2 progeny, the selection of genotypes carrying resistance to herbicides from the acetolactate synthase group of inhibitors was performed by spraying them with a herbicide from this group. In the surviving plants, identification of genotypes carrying the inactivated BnSpPS orthologs was performed.

The maternal rape line was maintained by spraying with 4-oxo-6-octadecane-pyran-2-olate in a dose of 2-5 kg.ha⁻¹ in the form of a water emulsion in the phase in which pollen tetrads are formed. Given the fact that the rape plant flowers continuously for approximately 30 days, a further three doses were applied every 14 days after the first treatment. The biological effectiveness of 4-oxo-6-octadecane-pyran-2-olate is tested by protecting parts of the plants with an insulating construction before spraying. The insulating construction is then applied to the plant during flowering. As a control variant, identical procedure is performed with plants in which the BnSpPS orthologues have not been inactivated (the rape plant variety Cortes). The degree of sterility and the degree of restoration of fertility is expressed by the percent of seeds created by the maternal line (BnSt13) compared to the untreated control variant (Cortes). The degree of potential toxicity is expressed by the reduction of the number of seeds in the treated (sprayed) control variant compared to the untreated (insulated) control variant, expressed in %. The summarized results of the testing are shown in the following table:

| | Seed weight per plant | | | | | |
|---|---|---|---|---|---|---|
| Dose of oxo-6-octadecane-pyran-2-olate | 2 kg.ha⁻¹ | | 3.5 kg.ha⁻¹ | | 5 kg.ha⁻¹ | |
| Line | Insulation | Spraying | Insulation | Spraying | Insulation | Spraying |
| Cortes | 24 | 23 | 24 | 22 | 24 | 20,5 |
| BnSt13 | 0 | 23 | 0 | 21,8 | 0 | 20 |
| Sterility (%) | 100 | | 100 | | 100 | |
| Restoration of fertility (%) | 96 | | 91 | | 83 | |
| Toxicity (%) | 4 | | 9 | | 17 | |

The inactivation of BnSpPS orthologs on both genomes, therefore, resulted in an induction of 100% sterility; treatment with oxo-6-octadecane-pyran-2-olate resulted in an 83-96% restoration of fertility, whereby toxicity manifested itself at a level of 4-17%.

Production of F1 hybrid rape seed: the seed from the maternal line is mixed with the seed from the paternal line in a ratio of 95:5, and sown. At the time of flowering, the sterile maternal line is pollinated by the fertile paternal line. After the flowering ends, the vegetation is treated with herbicide from the acetolactate synthase inhibitor group. The herbicide-sensitive paternal line is killed, and the maternal line which produces the pure fertile hybrid F1 seed stays in the vegetation.

The testing of the heterotic effect of the created F1 rape seed took place using standard experimental methodology. The F1 seed was, together with control varieties (marked *), sown in micro-parcels in two repetitions and in two localities. The harvested seed was weighed, and the results were statistically analysed. The summarized results of the testing are shown in the following table:

| | Yield % | | |
|---|---|---|---|
| | Lu 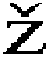 any | Stupice | Average |
| F1 Cortes x BnSt13 | 135 | 131 | 133 |
| Cortes* | 102 | 103 | 102.5 |
| Adriana* | 98 | 97 | 97.5 |

### Industrial applicability

The method of producing a hybrid F1 seed is useful especially in the production of seeds. Hybrid F1 seeds generally show a 5-50 % higher yield.

### References:

Bovina R, Talamè V, Silvio S, Sanguineti M.C., Trost P., Sparla F., Tuberosa R. (2011). Starch metabolism mutants in barley: A TILLING approach. Plant Genetic Resources 9: 170-173.
Cisar, G. and Cooper D.B. (2002): Hybrid wheat. FAO Plant Production and Protection Series (URI:http://www.fao.org/docrep/006/Y4011E/y4011e0c.htm).
EP 2 294 204 B1, Process of producing male sterile monocotyledonous plants.
Gilchrist E. J., Sidebottom CH. H. D., Koh Ch. S., MacInnes T., Sharpe A.G., Haughn G.W. (2013). A Mutant Brassica napus (Canola) Population for the Identification of New Genetic Diversity via TILLING and Next Generation Sequencing. PLoS ONE 8: e84303.
Lee H., Rustgi S., Kumar N., Burke I., Yenish J.P., Gill K.S., von Wettstein D., Ullrich S.E. (2011). Single nucleotide mutation in the barley acetohydroxy acid synthase (AHAS) gene confers resistance to imidazolinone herbicides. Proc Natl Acad Sci USA 108(21): 8909-13. doi: 10.1073/pnas.1105612108.
Swanson E.B., Herrgesell M.J., Arnoldo M., Sippell D.W., Wong R.S.C. (1989). Microspore mutagenesis and selection: Canola plants with field tolerance to the imidazolinones. Theoretical and Applied Genetics 78(4): 525-530.
Uauy C, Paraiso F, Colasuonno P., Tran R.K., Tsai H., Berardi S., Comai L., Dubcovsky J. (2009). A modified TILLING approach to detect induced mutations in tetraploid and hexaploid wheat. BMC Plant Biology 9: 115 doi:10.1186/1471-2229-9-115.

## Claims

1. A method of producing hybrid F1 seed of an angiosperm plant, **characterized in that** it contains the following steps:
- a maternal line is prepared by inactivation of a gene involved in the synthesis of sporopollenin and by mutation of acetolactate synthase gene resulting in resistance to herbicides of the acetolactate synthase inhibitor group;
- a paternal line is prepared, which is a fertile line without resistance to herbicides of the acetolactate synthase inhibitor group;
- subsequently, hybrid F1 seeds are produced by mixing the seed of the sterile herbicide-tolerant maternal line and the fertile herbicide-sensitive paternal line in a 95:5 ratio and by subsequent sowing this mixture of seeds of the maternal line and paternal line; and
- after the flowering period of the vegetation of mixture of the maternal line and paternal line, the plants are treated with a herbicide of the acetolactate synthase inhibitor group, thereby killing the herbicide-sensitive paternal line plants and leaving the herbicide-resistant maternal line plants producing pure fertile hybrid F1 seed;
- whereas the sterile maternal line is maintained by spraying with 4-oxo-6-octadecane-pyran-2-olate in the phase in which pollen tetrads are formed.

2. The method according to claim 1, wherein the inactivation is carried out by chemical mutagenesis.

3. The method according to claim 1, wherein the mutation of acetolactate synthase gene is carried out by chemical mutagenesis.

4. The method according to claim 1, wherein spraying with 4-oxo-6-octadecane-pyran-2-olate is performed in a dose of 2-5 kg.ha⁻¹ of 4-oxo-6-octadecane-pyran-2-olate.

5. The method according to claim 1, wherein the genes involved in the synthesis of sporopollenine are selected from TaSpPS and its homologs in wheat, HvSpPS in barley, BnSpPS1, BnSpPS2, BnSpPS3 and their homologs in rape, or orthologs and/or homologs of these genes in other angiosperm plants.

6. The method according to claim 1, wherein the angiosperm plant is selected from wheat, barley and rape.

## Amended claims

### Amended claims in accordance with Rule 137(2) EPC.

1. A method of producing hybrid F1 seed of an angiosperm plant, **characterized in that** it contains the following steps:
- a maternal line is prepared by inactivation of a gene involved in the synthesis of sporopollenin, selected from TaSpPS and its homologs in wheat, HvSpPS in barley, BnSpPS1, BnSpPS2, BnSpPS3 and their homologs in rape, or orthologs and/or homologs of these genes in other angiosperm plants, and by mutation of acetolactate synthase gene resulting in resistance to herbicides of the acetolactate synthase inhibitor group;
- a paternal line is prepared, which is a fertile line without resistance to herbicides of the acetolactate synthase inhibitor group;
- subsequently, hybrid F1 seeds are produced by mixing the seed of the sterile herbicide-tolerant maternal line and the fertile herbicide-sensitive paternal line in a 95:5 ratio and by subsequent sowing this mixture of seeds of the maternal line and paternal line; and
- after the flowering period of the vegetation of mixture of the maternal line and paternal line, the plants are treated with a herbicide of the acetolactate synthase inhibitor group, thereby killing the herbicide-sensitive paternal line plants and leaving the herbicide-resistant maternal line plants producing pure fertile hybrid F1 seed;
- whereas the sterile maternal line is maintained by spraying with 4-oxo-6-octadecane-pyran-2-olate in the phase in which pollen tetrads are formed.

2. The method according to claim 1, wherein the inactivation is carried out by chemical mutagenesis.

3. The method according to claim 1, wherein the mutation of acetolactate synthase gene is carried out by chemical mutagenesis.

4. The method according to claim 1, wherein spraying with 4-oxo-6-octadecane-pyran-2-olate is performed in a dose of 2-5 kg.ha⁻¹ of 4-oxo-6-octadecane-pyran-2-olate.

5. The method according to claim 1, wherein the angiosperm plant is selected from wheat, barley and rape.
